# EUROPEAN PATENT APPLICATION

(11) **EP 1 321 522 A1**
(43) Date of publication of application: **25.06.2003**
(21) Application number: 01403290.8
(22) Date of filing: 18.12.2001
(51) Int. Cl.: C12N 15/11, A61K 39/395, A61P 9/10

(54) **Promoting cell survival by inhibiting BARD1 apoptotic activity**

(71) Applicant: UNIVERSITE DE GENEVE, CH-1211 Geneve 4 (CH)
(72) Inventor: Irminger, Irmgard, 1207 Geneve (CH); Krause, Karl-Heinz, 1206 Genéve (CH)
(74) Representative: Bernasconi, Jean Raymond

(57) **Abstract**

The present invention provides a method for promoting cell survival, which method comprises inhibiting BARD1 activity. This method may be performed by contacting a cell with an agent that inhibits BARD1 activity. Such agent may be an agent that inhibits BARD 1 expression, such as a BARD1 antisense nucleic acid. The method of the invention is especially useful for alleviating damage in cells subjected to ischemia.

## Description

The invention relates to a method for promoting cell survival, by inhibiting BARD1 apoptotic activity in cells in need of such treatment.

BARD1 (BRCA1-associated RING domain 1) is described as a nuclear protein that associates with the breast cancer susceptibility gene product BRCA1 (Wu et al., (1996) ; Miki et al., (1994)). The two proteins interact *in vivo* and *in vitro* through their respective RING finger domains (Wu et al., (1996) ; Meza et al., (1999) ; Scully et al., (1997)).

As mutations in the BRCA1 RING finger domain (Bienstock et al., (1996)) lead to increased cancer susceptibility, BARD1 was thought to be implicated in BRCA1-mediated tumor suppression. Consistent with this hypothesis, BARD1 mutations were found in breast, ovarian, and uterine tumors (Thai et al., (1998)), and reduced expression of BARD1 is associated with spontaneous breast cancer cases (Yoshikawa et al., (2000)). The *in vitro* repression of BARD1 in murine mammary gland epithelial cells led to a pre-malignant phenotype and genomic instability (Irminger-Finger et al., (1998)). The BARD1/BRCA1 complex was shown to associate with the polyadenylation factor CstF-50 (Kleiman et al., (1999)), presumably to prevent RNA processing at sites of DNA repair (Kleiman and Manley, (2001)). The BRCA1-BARD1 heterodimeric RING finger complex was recently shown to possess ubiquitin ligase activity *in vitro* (Hashizume et al., (2001)).

However, BARD1 and BRCA1 are not consistently co-expressed in different tissues (Irminger-Finger et al., (1998)), suggestive of the existence of independent functions for both genes. Of particular interest in this context is the previously observed high expression level of BARD1 but undetectable BRCA1 concentrations in mouse uterus during the oestrous phase (Irminger-Finger et al., (1998)), i.e. the moment of the oestrous cycle when massive cell death (in part through apoptosis) of the endometrium occurs (Ferenczy, (1993)).

A highly immunogenic cleavage product of BARD1 in apoptotic bodies has also been identified, that could contribute to the antitumoral response mediated by apoptotic bodies (Gautier et al., (2000)).

Yet, the involvement of BARD1 in cell survival had not been investigated so far.

The inventors have observed that BARD1 is upregulated upon *in vitro* or *in vivo* stress. In the light of these results, the present invention provides a new therapeutic pathway to promote cell survival.

The inventors more particularly propose to inhibit BARD1 activity in cells in need of such treatment, especially cells subjected to ischemia.

The present invention provides a method for promoting cell survival, which method comprises inhibiting BARD1 activity.

This method may be performed *in vivo* or *ex vivo,* by contacting a cell with an agent that inhibits BARD1 activity.

Another subject of the invention is the use of an agent that inhibits BARD1 activity for the preparation of a medicament useful for promoting cell survival.

The agent that inhibits BARD1 activity may be an agent that inhibits BARD 1 expression, such as a BARD1 antisense nucleic acid.

In another embodiment, the agent that inhibits BARD1 activity is an agent that inhibits BARD1 protein functional properties, especially BARD1 interaction with p53. Such agents include anti-BARD1 inhibitory antibodies, as well as agents that can be identified by screening assays.

The agent that inhibits BARD1 activity may be formulated in a pharmaceutical composition that is useful for alleviating damage in cells subjected to ischemia, in particular ischemia that results from a stroke or heart failure.

The present invention further encompasses co-inhibition of BARD1 and p53 activities. For that purpose the agent that inhibits BARD1 activity may be associated with an agent that inhibits p53 activity.

Various methods can be contemplated in that respect.

A particular subject of the invention is a nucleic acid that comprises a BARD1 antisense sequence and a p53 antisense sequence.

Another subject of the invention is a pharmaceutical composition containing either such a nucleic acid (e.g. in the form of a vector), or an association of
i) a BARD1 antisense nucleic acid, and
ii) a p53 antisense nucleic acid,
in a pharmaceutically acceptable carrier.

The invention further provides a kit comprising
- in a first container, a pharmaceutical composition containing a BARD1 antisense sequence in a pharmaceutically acceptable carrier; and
- in a second container, a pharmaceutical composition containing comprising a p53 antisense sequence in a pharmaceutically acceptable carrier.

In a particular embodiment of the invention, the cell killing is enhanced by stimulating BRCA1 activity or expression while inhibiting BARD1 activity. Accordingly, the agent that inhibits BARD1 activity may be associated with an agent that stimulates BRCA1 expression, or with a BRCA1 expressing nucleic acid.

In that respect, the present invention is also directed to a pharmaceutical composition comprising :
i) a BARD1 antisense nucleic acid ; and
ii) a BRCA1 encoding nucleic acid ;
in association with a pharmaceutically acceptable carrier.

The invention further provides a kit comprising :
- in a first container, a pharmaceutical composition containing a BARD1 antisense nucleic acid in a pharmaceutically acceptable carrier;
- in a second container, a pharmaceutical composition comprising an expression vector that comprises a BRCA1 encoding nucleic acid operatively associated with expression control sequences.

### BARD1 activity :

In the context of the present invention, *"inhibiting BARD1 activity"* means either directly inhibiting BARD1 functional properties or inhibiting the endogenous BARD1 expression (i.e. transcription or translation of BARD1 coding sequence). BARD1 functional properties refer to the biological functions of BARD1, including its ability to interact with p53, hence its proapoptotic property, or control of genetic instability. BARD1 interferes with the rapid turnover of the p53 protein. It is suggested that BARD1 interferes with mdm2 binding to p53, thus with p53 ubiquitination.

A reduction of at least 30 %, or 50 %, more preferably 80 % or up to 100 % of the constitutive BARD1 level (with respect to the BARD1 amount or activity) is particularly advantageous.

### Ischemia :

In a preferred embodiment, cells, survival of which is desired, are cells that undergo a stress, *e.g.* in ischemia or hypoxia conditions. The inventors have observed that ischemia induced cells death is associated with BARD1 upregulation in the brain after stroke. A similar phenomenon is expected after heart failure.

Tissues deprived of blood and oxygen undergo ischemic necrosis or infarction with possible irreversible organ damage. Cerebral ischemia results from decreased blood and oxygen flow implicating one or more of the blood vessels of the brain. In cerebral ischemia, the individual suffers a stroke with sudden development of a focal neurologic deficit and, in most cases, some degree of brain damage. The decreased blood flow may be due to, for example, an occlusion such as a thrombus or embolus, vessel rupture, sudden fall in blood pressure, change in the vessel lumen diameter due to atherosclerosis, trauma, aneurysm, developmental malformation, altered permeability of the vessel wall or increased viscosity or other quality of the blood. Decreased blood flow may also be due to failure of the systemic circulation and severe prolonged hypotension. Ischemic necrosis of the spinal cord may result in sensory or motor symptoms or both that can be referred to cervical, thoracic or lumbar levels of the spine. Ischemic heart disease results from an imbalance between myocardial oxygen supply and demand. In ischemic heart disease, the individual suffers angina pectoris, acute myocardial infarction or sudden death. The imbalance may be caused by, for example, atherosclerotic obstruction of one or more large coronary arteries, nonatheromatous coronary obstructive lesions such as embolism, coronary ostial stenosis associated with luetic aortitis, coronary artery spasm, congenital abnormalities of the coronary circulation, increased myocardial oxygen demands exceeding the normal supply capabilities as in severe myocardial hypertrophy, reduction in the oxygen carrying capacity of the blood such as in anemia, or as a consequence of inadequate cardiac perfusion pressure due to hypotension from any cause.

Promoting cell survival according to the method of the invention is particularly helpful when a stroke has occurred, to alleviate damage caused by ischemia and avoid many serious resulting after-effects.

Prevention of these damaging effects in asymptomatic patients (i.e. patients without evidence of stroke) is further contemplated.

These embodiments are described in greater detail hereafter.

Inhibition of BARD1 activity particularly allows to ameliorate or inhibit damage caused by a stroke. A stroke is the acute neurologic injury caused by one of several pathologic processes involving the blood vessels of the brain. The pathologic process may be intrinsic to the vessel itself such as in arteriosclerosis, or may originate from a remote location such as an embolus, or may result from decreased perfusion or increased blood viscosity with inadequate cerebral blood flow, or may result from the rupture of a vessel in the subarachnoid space or intracerebral tissue.

The main causes of ischemic stroke are thrombosis, vasoconstriction and embolism. Diagnosis of a stroke can be readily made by one of ordinary skill in the art. Signs of stroke include paralysis, slurred speech, general confusion, impairment of gait, cortical sensory loss over toes, foot and leg and urinary incontinence, to name just a few. The diagnosis can be confirmed by cerebral angiography and by a computed axial tomography (CT) scan of the brain.

Very often a stroke is caused by a cerebral embolism, the likelihood of which can frequently be predicted. In these cases, the BARD1 inhibition can be contemplated prophylactically to prevent or lessen the amount of brain tissue injured during such an event. Many types of heart disease including cardiac arrhythmias or diseases due to cardiac structural abnormalities may produce cerebral emboli. Atrial fibrillation from any cause, including rheumatic valvular disease, may result in emboli being produced which can migrate into the arteries of the brain. Emboli formation and migration can occur as a result of arteriosclerotic cardiovascular disease and myocardial infarction. Emboli formation is also a definite risk for intracardiac surgery and prosthetic valve replacement. Heart bypass surgery and angioplasty can result in the formation of microemboli which can migrate into the arteries of the brain and cause a series of occlusions in a number of arteries, resulting in mental impairment. Cerebral embolism is also the principal complication in the transplant of artificial hearts. Furthermore, the overall risk of stroke after any type of general surgery is 0.2 to 1 percent. The vegetations of acute and subacute bacterial endocarditis can give rise to emboli which can occlude a major intracranial artery. Thus, when these disease states or surgical procedures are planned or are happening, BARD1 inhibition can be contemplated to prevent brain damage due to any resultant emboli and stroke.

Inhibition of BARD1 can also allow to ameliorate or prevent ischemic necrosis of the spinal cord. The ischemia may be caused by an endarteritis of surface arteries leading to thrombosis. Atherosclerotic thrombosis of the aorta or dissecting aortic aneurysms may cause infarction of the spinal cord (myelomalacia) by occluding nutrient arteries at cervical, thoracic or lumbar levels, as can paralysis during cardiac surgery requiring clamping of the aorta for more than 30 minutes and aortic arteriography. Infarctive or hemorrhagic vascular lesions of the spinal cord (hematomyelia) may result in the sudden onset of symptoms referable to sensory or motor or both spinal tract lesions.

Inhibition of BARD1 is further useful can also be administered to ameliorate or inhibit damage caused by ischemic heart disease. Ischemic heart disease is a general term for a spectrum of diseases of diverse etiology caused by an imbalance between oxygen supply and demand. The usual cause of the imbalance is atherosclerotic obstruction of large coronary arteries, leading to an absolute decrease in coronary artery blood flow. An imbalance may also result from nonatheromatous coronary obstructive lesions such as embolism, coronary ostial stenosis associated with luetic aortitis, coronary artery spasm, or very uncommonly an arteritis of the coronary vessels. The imbalance may also be due to congenital abnormalities of the coronary circulation, an increase in myocardial oxygen demands exceeding the supply capabilities in a normal coronary circulation, a diminished oxygen-carrying capacity of the blood such as in anemia or in the presence of carboxyhemoglobin (*e.g.*, due to cigarette or cigar smoking), or as a consequence of inadequate perfusion pressure due to hypotension. When ischemic events are transient, they may be associated with angina pectoris; if prolonged, they can lead to myocardial necrosis and scarring with or without the clinical picture of acute myocardial infarction.

Ischemic heart disease may be readily diagnosed by one skilled in the art. There may be predictive changes in the electrocardiogram, since ischemia alters electrical properties of the heart. Such changes include inversion of the T wave and displacement of the ST segment. Another important consequence of myocardial ischemia is electrical instability leading to ventricular tachycardia or ventricular fibrillation. Stress tests and coronary arteriography may also provide diagnostic information. These diagnostic test results may determine the need for BARD1 inhibition.

Since ischemic heart disease is usually asymptomatic until the extent of coronary artery blockage is well advanced, preventative measures to control risk factors and life style patterns associated with the disease are also recommended. In patients in the symptomatic phase of the disease, meticulous attention to life patterns or risk factors must be given in an attempt to promote lesion regression or at least prevent progression. Risk factors include a positive family history of ischemic heart disease, diabetes, hyperlipidemia, hypertension, obesity and cigarette smoking. Life patterns include sedentary lifestyle, psychosocial tension and certain personality traits.

Inhibition of BARD1 may be contemplated for asymptomatic individuals having one or more risk factors and/or life style patterns, or for individuals already in the symptomatic phase of ischemic heart disease to reduce or prevent disease progression. Additionally, inhibition of BARD1 may be helpful for the following patients: those having careers that involve the safety of others (*e.g.* commercial airline pilots) and that present with questionable symptoms, suspicious or positive noninvasive test results, and in whom there are reasonable doubts about the state of the coronary arteries; males who are 45 or older and females who are 55 or older who will undergo valve replacement and who may or may not have clinical evidence of myocardial ischemia; and those at high risk after myocardial infarction because of the recurrence of angina, heart failure, frequent ventricular premature contractions, or signs of ischemia in the stress test, to name just a few. Inhibition of BARD1 activity is contemplated either separately or in combination with administering cardiac drugs such as nitrates, beta-adrenergic blockers, calcium channel antagonists and/or aspirin and either separately or in combination with fibrinolytic drugs such as tissue plasminogen activator (tPA), streptokinase and urokinase. Inhibition of BARD1 activity may prolong life and/or reduce or eliminate the need for invasive procedures such as coronary arteriography and coronary artery bypass grafting.

Efficacy of BARD1 inhibition treatment may be evaluated using noninvasive clinical imaging methods such as magnetic resonance imaging (MRI) of the affected region to determine the size of the damaged area. In cerebral ischemia, it is also possible to assess neurologic deficit by performance on behavioral tests such as cognitive recognition or memory function.

### Other applications:

Other applications of BARD1 repression to permit cell survival include apoptotic loss of spermacytes during spermatogenesis.

This indication is based on various observations including observation of BARD1 expression in rat testis. The inventors more particularly revealed BARD1 expression in pachitaen spermatocytes by staining with an anti-BARD1 antibody. The inventors observed that first, BARD1 protein was expressed in spermatogonia, and second, that later stages showed apoptotic spermatocytes phagocytosed by Sertolli cells.

### p53 co-inhibition :

In a particular embodiment, the present invention contemplates inhibiting p53 activity, in addition to inhibiting BARD1 activity.

The gene for the nuclear phosphoprotein p53 is the most commonly mutated gene yet identified in human cancers (Vogelstein, (1990)). Missense mutations occur in tumors of the colon, lung, breast, ovary, bladder, and several other organs.

In the context of the present invention, *"inhibiting p53 activity"* means either directly inhibiting p53 functional properties or inhibiting the endogenous p53 expression (i.e. transcription or translation of p53 coding sequence). P53 functional properties refer to the biological functions of p53, including its ability to interact with BARD1, or its proapoptotic property.

A reduction of at least 30 %, or 50 %, more preferably 80 % or up to 100 % of the constitutive p53 level (with respect to the p53 amount or activity) is particularly advantageous.

Inhibiting p53 activity is intended to enhance the inhibition of BARD1 apoptotic function, since BARD1 proapoptotic effect occurs through p53 pathway.

For example, this co-inhibition can be obtained by simultaneously or sequentially administering a composition that inhibits BARD1 and a composition that inhibits p53. Alternatively, an agent that inhibits BARD1 can be mixed to an agent that inhibits p53 in the same pharmaceutical composition. When antisense nucleic acids are used, cells can be either co-transfected with two types of vectors (one transferring BARD1 antisense sequence, the other transferring p53 antisense sequence) or transfected with a single type of vector that is designed to transfer both BARD1 and p53 antisense sequences. All the above embodiments are referred herein as a "therapeutic association".

Means for inhibiting p53 activity are similar to the means described with respect to the inhibition of BARD1 activity. For this reason, they are not described extensively herein, for one skilled in the art will know how to adapt the description below to achieve p53 inhibition. P53 inhibiting substances could act transcriptionally or preferably on the protein level.

Inhibition of p53 could also be induced by upregulation of mdm2 and E6AP, two ubiquitin ligases known to interact and ubiquitinate p53 thus tagging p53 for degradation.

Furthermore, many references are available in that prospect. P53 inhibiting substances could act transcriptionally, or, more likely, on the protein level.

### BRCA1/BARD1 ratio :

The BRCA1 gene has been cloned in 1994 (Miki et al.). It encodes an 1863 aminoacids protein with an apparent molecular mass of 220 kDa (Chen et al., (1995) ; Scully et al., (1996)). Frequent loss of the wild-type allele in tumors of mutation carriers suggests that BRCA1 acts as a tumor suppressor gene. Several lines of evidence indicate that BRCA1 would have a role in DNA damage-dependent replication checkpoint response (Scully et al., (1997) ; Zhong et al. (1999) ; Wang et al (2000)). Accordingly, its ability to bind directly DNA structures may be important for its role in DNA repair (Paull et al., (2001)). BRCA1 would have a role in transcription-coupled repair of oxidative damages (Gowen et al., (1998) ; Abbott et al., (1999)).

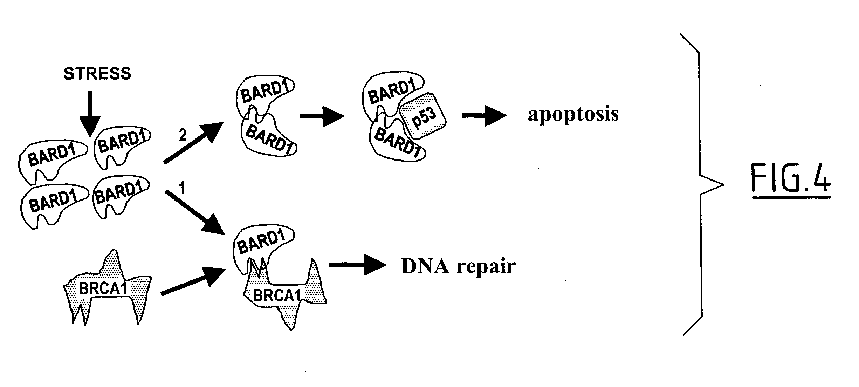

Based on the BRCA1-dependent BARD1 functions described previously and on the BRCA1-indepedent BARD1 function described herein, the inventors propose a "dual mode of action" model of BARD1 function (Fig. 4). In the survival mode, BARD1 acts in a heterodimeric complex with BRCA1 and is involved in DNA repair and cell survival. In the death mode, BARD1 acts independently of BRCA1, and induces apoptosis. Thus, according to this model, the ratio of BRCA1 and BARD1 expressed in a given cell would determine cell fate; a high BRCA1/BARD1 ratio would lead to DNA repair and survival, while a low ratio would lead to cell death. This model is compatible with the observed expression of BARD1, but not BRCA1 under conditions where cell death occurs, *e.g*. stroke, and estrus phase of the uterine cycle.

In the context of the present invention, it is therefore advantageous to stimulate BRCA1 activity or expression while inhibiting BARD1 activity or expression.

This can be achieved by various methods that one skilled in the art is familiar with.

In a particular embodiment cells can be cotransfected with a BARD1 antisense nucleic acid and a BRCA1 encoding nucleic acid. Such cotransfection can be preferably accomplished by using two types of vectors (one transferring BARD1 antisense sequence, the other transferring BRCA1 encoding sequence, operately associated with expression control sequences).

Introducing exogenous BRCA1 protein or agents that stimulate BRCA1 endogenous expression can be useful too.

### In vivo and ex vivo methods :

The method of the invention can be performed *in vivo* or *ex vivo.* In the sense of the present invention, *"ex vivo methods"* refer to methods wherein at least one step is carried out *ex vivo.*

In the *in vivo* methods, promotion of cell survival is achieved by administering a patient with an agent that inhibits BARD1 activity. The agent may be administered by any route, especially directly into the site of cells in need of such treatment.

In the *ex vivo* methods promotion of cell survival is achieved by contacting the cells with an agent that inhibits BARD1 activity. The cells may be obtained from biopsies or biological samples from patients in need of such treatment. They are returned or grafted to the patient after the action of the BARD1 inhibitor agent. This may be particularly indicated to treat germinal cells, *e.g.* in patients wherein an excessive apoptotic loss of spermatocytes is observed. Another application is to engineer cells to be used in stem cell therapy in a way that delimits BARD1 upregulation upon stress. Cells capable to differentiate into a specific cell type can be treated according to the present invention, so that they are rendered less responsive or non-responsive to stress. This is especially useful to strengthen a patient's heart that undergoes ischemic heart disease for instance. Stem cells treated *ex vivo* to differentiate into heart cells that are more resistant to an ischemic stress can then be grafted to the patient in need of such treatment (Watt and Hogan 2000).

### Inhibition of BARD1 transcription :

According to one embodiment of the invention, inhibition of BARD1 activity is achieved by inhibiting BARD1 transcription.

One skilled in the art can select various strategies therefor.

For instance, antisense nucleic acids, including ribozymes, may be used to inhibit expression of BARD1 protein.

When transfected into a cell via a viral vector antisense nucleic acids can be stably integrated and provide a long-term inhibition. Alternatively, antisense oligonucleotides can be directed administered and provides a transient inhibition. In some cases, such as a stroke, this method can be sufficient to guarantee the survival of a majority cells during a few hours after stroke.

An *"antisense nucleic acid"* is a single stranded nucleic acid molecule which, on hybridizing under cytoplasmic conditions with complementary bases in an RNA or DNA molecule, inhibits the latter's role. If the RNA is a messenger RNA transcript, the antisense nucleic acid is a countertranscript or mRNA-interfering complementary nucleic acid. As presently used, "antisense" broadly includes RNA-RNA interactions, RNA-DNA interactions, ribozymes and RNase-H mediated arrest. Antisense nucleic acid molecules can be encoded by a recombinant gene for expression in a cell (*e.g.,* U.S. Patent No. 5,814,500 ; U.S. Patent No. 5,811,234), or alternatively they can be prepared synthetically (*e.g.,* U.S. Patent No. 5,780,607).

*"BARD1 antisense"* nucleic acids are preferably designed to be specifically hybridizable with a BARD1 encoding sequence, *e.g.* the human sequence available on GenBank/EMBL (access number : HSU 76638 or AF 038042).

A nucleic acid molecule is *"hybridizable"* to another nucleic acid molecule, such as a cDNA, genomic DNA, or RNA, when a single stranded form of the nucleic acid molecule can anneal to the other nucleic acid molecule under the appropriate conditions of temperature and solution ionic strength *(see* Sambrook *et al.,* 1989). The conditions of temperature and ionic strength determine the "stringency" of the hybridization. For preliminary screening for homologous nucleic acids, low stringency hybridization conditions, corresponding to a Tm (melting temperature) of 55°C, can be used, *e.g.,* 5x SSC, 0.1 % SDS, 0.25 % milk, and no formamide ; or 30 % formamide, 5x SSC, 0.5 % SDS). Moderate stringency hybridization conditions correspond to a higher Tm, *e.g.,* 40 % formamide, with 5x or 6x SCC. High stringency hybridization conditions correspond to the highest Tm, *e.g.,* 50 % formamide, 5x or 6x SCC. SCC is a 0.15 M NaCI, 0.015 M Na-citrate. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived *(see* Sambrook *et al.*, 1989, 9.50-9.51). For hybridization with shorter nucleic acids, *i.e.,* oligonucleotides, the position of mismatches becomes more important, and the length of the oligonucleotide determines its specificity (*see* Sambrook *et al.,* 1989, 11.7-11.8). A minimum length for a hybridizable nucleic acid is at least about 10 nucleotides ; preferably at least about 15 nucleotides ; and more preferably the length is at least about 20 nucleotides.

In a specific embodiment, the term *"standard hybridization conditions"* refers to a Tm of 55°C, and utilizes conditions as set forth above. In a preferred embodiment, the Tm is 60°C. In a more preferred embodiment, the Tm is 65°C. In a specific embodiment, "high stringency" refers to hybridization and/or washing conditions at 68°C in 0.2 X SSC, at 42°C in 50 % formamide, 4 X SSC, or under conditions that afford levels of hybridization equivalent to those observed under either of these two conditions.

Antisense therapy usually makes use of a vector (*e.g.* a plasmid) that carries the antisense sequence.

The use of such a vector indeed makes it possible to improve the administration of the nucleic acid into the cells to be treated, and also to increase its stability in the said cells, which makes it possible to obtain a durable therapeutic effect. Furthermore, it is possible to introduce several nucleic acid sequences into the same vector, which also increases the efficacy of the treatment.

The vector used may be of diverse origin, as long as it is capable of transforming animal cells. In a preferred embodiment of the invention, a viral vector is used which can be chosen from adenoviruses, retroviruses, adeno-associated viruses (AAV), herpes virus, cytomegalovirus (CMV), vaccinia virus and the like. Vectors derived from adenoviruses, retroviruses or AAVs incorporating heterologous nucleic acid sequences have been described in the literature (Akli et al. (1993) 224; Stratford-Perricaudet et al. (1990) ; Levrero et al., (1991) ; Le Gal la Salle et al., (1993) ; Roemer et al. (1992) ; Dobson et al., (1990) ; Chiocca et al. (1990) ; Miyanohara et al. (1992)).

The present invention therefore also relates to any recombinant virus comprising, inserted into its genome, a BARD1 antisense nucleic acid sequence as defined before, optionally accompanied with a p53 antisense nucleic acid sequence.

Advantageously, the recombinant virus according to the invention is a defective virus. The term "*defective virus*" designates a virus incapable of replicating in the target cell. Generally, the genome of the defective viruses used within the framework of the present invention is therefore devoid of at least the sequences necessary for the replication of the said virus in the infected cell. These regions can either be removed (completely or partially), or rendered non-functional, or substituted by other sequences and especially by the BARD1 nucleic acid of the invention. Preferably, the defective virus nevertheless conserves the sequences of its genome which are necessary for the encapsulation of the viral particles.

It is particularly advantageous to use the BARD1 nucleic acid sequences in a form incorporated in an adenovirus, an AAV or a defective recombinant retrovirus.

As regards adenoviruses, various serotypes exist whose structure and properties vary somewhat, but which are not pathogenic for man, and especially non-immunosuppressed individuals. Moreover, these viruses do not integrate into the genome of the cells which they infect, and can incorporate large fragments of exogenous DNA. Among the various serotypes, the use of the type 2 or 5 adenoviruses (Ad2 or Ad5) is preferred within the framework of the present invention. In the case of the Ad5 adenoviruses, the sequences necessary for the replication are the E1A and E1B regions.

Other convenient vectors would be retroviral HIV derived vectors with high transduction efficiency.

The defective recombinant viruses of the invention can be prepared by homologous recombination between a defective virus and a plasmid carrying, *inter alia,* the nucleotide sequence as defined above (Levrero et al. (1991) ; Graham (1984)). The homologous recombination is produced after co-transfection of the said viruses and plasmid into an appropriate cell line. The cell line used should preferably (i) be transformable by the said elements, and (ii), contain sequences capable of complementing the part of the genome of the defective virus, preferably in integrated form so as to avoid the risks of recombination. As example of a line which can be used for the preparation of defective recombinant adenoviruses, there may be mentioned the human embryonic kidney line 293 (Graham et al. (1977)) which contains especially, integrated into its genome, the left part of the genome of an Ad5 adenovirus (12%). As example of a line which can be used for the preparation of defective recombinant retroviruses, there may be mentioned the CRIP line (Danos et al. (1988)).

Then the viruses which have multiplied are recovered and purified according to conventional molecular biology techniques.

Targeted gene delivery is described in International Pat. Publication WO 95/28 494, published October 1995.

Alternatively, the vector can be introduced *in vivo* by lipofection. For the past decade, there has been increasing use of liposomes for encapsulation and transfection of nucleic acids *in vitro.* Information regarding liposomes is provided in the *"pharmaceutical composition"* section of the present application as well.

Synthetic cationic lipids designed to limit the difficulties and dangers encountered with liposome mediated transfection can be used to prepare liposomes for *in vivo* transfection of a gene encoding a marker (Felgner, et. al. (1987) ; see Mackey et al. (1988)). The use of cationic lipids may promote encapsulation of negatively charged nucleic acids, and also promote fusion with negatively charged cell membranes (Felgner et al. (1989)). The use of lipofection to introduce exogenous genes into the specific organs *in vivo* has certain practical advantages. Molecular targeting of liposomes to specific cells represents one area of benefit. It is clear that directing transfection to particular cell types would be particularly advantageous in a tissue with cellular heterogeneity, such as pancreas, liver, kidney, and the brain. Lipids may be chemically coupled to other molecules for the purpose of targeting (see Mackey, et. al., supra). Targeted peptides, *e.g.* hormones or neurotransmitters, and proteins such as antibodies, or non-peptide molecules could be coupled to liposomes chemically.

It is also possible to introduce the vector *in vivo* as a naked DNA plasmid. Naked DNA vectors for gene therapy can be introduced into the desired host cells by methods known in the art, e.g., transfection, electroporation, microinjection, transduction, cell fusion, DEAE dextran, calcium phosphate precipitation, use of a gene gun, or use of a DNA vector transporter (see, *e.g.* Wu et al. (1992); Wu et al. (1988)).

In a particular embodiment, the invention provides a nucleic acid that comprises a BARD1 antisense sequence and a p53 antisense sequence. A subject of the invention is also a pharmaceutical composition containing such a nucleic acid, (*e.g.* in the form of a vector) comprising said nucleic acid in a pharmaceutically acceptable carrier. Another subject of the invention is a kit comprising in a first recipient, a pharmaceutical composition containing a BARD1 antisense sequence (*e.g.* in the form of a vector) in a pharmaceutically acceptable carrier ; and in a second recipient, a pharmaceutical composition containing a p53 antisense sequence (*e.g.* in the form of a vector) in a pharmaceutically acceptable carrier.

Antisense oligonucleotides can also be used to provide a transient BARD1 inhibition. For that purpose antisense, oligonucleotides, that are not part of a viral vector, can be administered to the cell by any means as described above.

As used herein, the term "oligonucleotide" refers to a nucleic acid, generally of at least 10, preferably at least 15, and more preferably at least 20 nucleotides, preferably no more than 100 nucleotides, that is hybridizable to a genomic DNA molecule, a cDNA molecule, or a mRNA molecule encoding a gene, mRNA, cDNA, or other nucleic acid of interest.

Oligonucleotides can be labelled, *e.g.,* with ³²P-nucleotides or nucleotides to which a label, such as biotin, has been covalently conjugated. Generally, oligonucleotides are prepared synthetically, preferably on a nucleic acid synthesizer. Accordingly, oligonucleotides can be prepared with non-naturally occurring phosphoester analog bonds, such as thioester bonds, etc.

Reversible short inhibition of transcription may also be useful, *e.g.* after a stroke or a heart failure, as described above. Such inhibition can be achieved by specific inhibitors of BARD1 transcription.

### Inhibition of BARD1 protein functions :

According to another embodiment of the invention, inhibition of BARD1 activity is achieved by inhibiting functional properties of BARD1 protein.

More particularly, one can target BARD1 apoptotic function, especially by interfering with BARD1 interaction with p53 binding, and/or regions of BARD1 protein that are involved in the apoptotic function.

This proapoptotic activity of BARD1 has been evidenced by the inventors and involves binding to and elevations of p53. This proapoptotic activity is sustained by a BRCT repeat (amino acids 592 to 765) containing C-terminal domain of the BARD1 protein. Whereas the BRCT domain and/or the upstream adjacent region of BARD1 are required for apoptosis induction, the deletion of the BRCA1 interacting BARD1 RING finger motif does not corroborate the apoptotic activity. BRCA1 is actually not required for, but partially counteracts, apoptosis induction by BARD1.

Agents useful to inhibit BARD1 protein functions include inhibitory antibodies, or compounds, such as small molecules, that can be identified by screening methods. These embodiments are described in greater detail below.

### Inhibitory antibodies:

Antibodies that inhibit BARD1 activity are of particular interest.

The term "*antibody*" in its various grammatical forms is used herein to refer to immunoglobulin molecules and immunologically active portions of immunoglobulin molecules, i.e., molecules that contain an antibody combining site or paratope. Exemplary antibody molecules are intact immunoglobulin molecules, substantially intact immunoglobulin molecules and portions of an immunoglobulin molecule, including those portions known in the art as Fab, Fab', F(ab')₂ and F(v).

Antibodies that inhibit the interaction of BARD1 with p53 are more particularly useful.

Whereas polyclonal antibodies, that are easy to obtain, may be used, monoclonal antibodies are preferred because they are more reproductible in the long run.

Procedures for raising polyclonal antibodies are well known. Typically, such antibodies can be raised by administering the BARD1 protein subcutaneously to New Zealand white rabbits which have first been bled to obtain pre-immune serum. The antigens can be injected at a total volume of 100 µl per site at six different sites. Each injected material will contain adjuvants with or without pulverized acrylamide gel containing the protein or polypeptide after SDS-polyacrylamide gel electrophoresis. The rabbits are then bled two weeks after the first injection and periodically boosted with the same antigen three times every six weeks. A sample of serum is then collected 10 days after each boost. Polyclonal antibodies are then recovered from the serum by affinity chromatography using the corresponding antigen to capture the antibody. This and other procedures for raising polyclonal antibodies are disclosed in Harlow et. al. (1988), which is hereby incorporated by reference.

A "*monoclonal antibody*" in its various grammatical forms refers to a population of antibody molecules that contain only one species of antibody combining site capable of immunoreacting with a particular epitope. A monoclonal antibody thus typically displays a single binding affinity for any epitope with which it immunoreacts. A monoclonal antibody may therefore contain an antibody molecule having a plurality of antibody combining sites, each immunospecific for a different epitope, *e.g.* a bispecific monoclonal antibody. Although historically a monoclonal antibody was produced by immortalization of a clonally pure immunoglobulin secreting cell line, a monoclonally pure population of antibody molecules can also be prepared by the methods of the present invention.

Laboratory methods for preparing monoclonal antibodies are well known in the art (see, for example, Harlow et al. (1988)). Monoclonal antibodies (Mabs) may be prepared by immunizing purified BARD1 protein isolated from any of a variety of mammalian species into a mammal, *e.g.* a mouse, rabbit, goat, human and the like mammal. The antibody-producing cells in the immunized mammal are isolated and fused with myeloma or heteromyeloma cells to produce hybrid cells (hybridoma). The hybridoma cells producing the monoclonal antibodies are utilized as a source of the desired monoclonal antibody.

While Mabs can be produced by hybridoma culture, the invention is not to be so limited. Also contemplated is the use of Mabs produced by an expressing nucleic acid cloned from a hybridoma of this invention. That is, the nucleic acid expressing the molecules secreted by a hybridoma of this invention can be transferred into another cell line to produce a transformant. The transformant is genotypically distinct from the original hybridoma but is also capable of producing antibody molecules of this invention, including immunologically active fragments of whole antibody molecules, corresponding to those secreted by the hybridoma. See, for example, U.S. Pat. No. 4,642,334 to Reading; PCT Publication No. WO 890099 to Robinson et al.; European Patent Publications No. 0239400 to Winter et al. and No. 0125023 to Cabilly et al. In addition, the literature provides methods for forming chimeric antibodies, humanized antibodies, single chain antibodies and the like variations on a basic immunoreactive antibody fragment. All of these are considered within the scope of the invention insofar as a class and specificity of antibody is disclosed and claimed, regardless of the precise variant structure that one skilled in the art may construct.

### Screening methods:

Inhibitor agents can be identified by screening methods, including biochemical and cellular *in vitro* assays.

Of particular interest is a process for screening substances for their ability to inhibit BARD1 activity, comprising the steps of providing a cell that expresses BARD1 and testing the ability of the substances to inhibit BARD1 activity, *e.g.* by blocking the interaction between BARD1 and p53, and/or by reducing BARD1 proapoptotic effect.

Test substances can be of any type, including natural or synthetic compounds or mixtures of compounds. The substance may be structurally defined or of unknown structure, *e.g.* in the form of a biological extract.

### Pharmaceutical compositions:

Antisense nucleic acids can be formulated in pharmaceutical compositions, for a topical, oral, parenteral, intranasal, intravenous, intramuscular, subcutaneous or intraocular administration and the like.

Preferably, the pharmaceutical compositions contain vehicles which are pharmaceutically acceptable for a formulation capable of being injected, optionally directly into the tumour to be treated. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses of nucleic acids (sequence or vector) used for the administration can be adapted as a function of various parameters, and in particular as a function of the mode of administration used, of the relevant pathology, of the nucleic acid to be expressed, or alternatively of the desired duration of treatment. Generally, with regard to recombinant viruses, the latter are formulated and administered in the form of doses of between 10⁴ and 10¹⁴ pfu/ml, and preferably 10⁶ to 10¹⁰ pfu/ml. The term pfu ("plaque forming unit") corresponds to the infectivity of a virus solution, and is determined by infecting an appropriate cell culture and measuring, generally after 48 hours, the number of plaques of infected cells. The techniques for determining the pfu titer of a viral solution are well documented in the literature.

Inhibitory agents, including inhibitory antibodies or other agents as defined above, are also advantageously used in the form of pharmaceutical compositions.

Examples of pharmaceutical formulations are provided hereafter.

Such pharmaceutical compositions comprise an effective amount of a BARD1 inhibitory agent in a pharmaceutically acceptable carrier or aqueous medium.

"*Pharmaceutically*" or "*pharmaceutically acceptable*" refer to molecular entities and compositions that do not produce an adverse, allergic or other untoward reaction when administered to an animal, or a human, as appropriate.

As used herein, "*pharmaceutically acceptable carrier*" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

The pharmaceutical forms suitable for injectable use include sterile aqueous solutions or dispersions ; formulations including sesame oil, peanut oil or aqueous propylene glycol ; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions of the active compounds as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

In terms of using peptide therapeutics as active ingredients, U.S. patents 4,608,251 ; 4,601,903 ; 4,599,231 ; 4,599,230 ; 4,596,792 and 4,572,770 provide useful information.

The preparation of more, or highly concentrated solutions for direct injection is also contemplated, where the use of DMSO as solvent is envisioned to result in extremely rapid penetration, delivering high concentrations of the active agents to a small tumor area.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCI solution and either added to 1000 ml of hypodermoclysis fluid or injected at the proposed site of infusion, (see for example, "Remington's Pharmaceutical Sciences" 15^{th} Edition, pages 1035-1038 and 1570-1580). Some variation in dosage will necessarily occur depending on the condition of the subject being treated. The person responsible for administration will, in any event, determine the appropriate dose for the individual subject.

The BARD1 inhibiting agents may be formulated within a therapeutic mixture to comprise about 0.0001 to 1.0 milligrams, or about 0.001 to 0.1 milligrams, or about 0.1 to 1.0 or even about 10 milligrams per dose or so. Multiple doses can also be administered.

In addition to the compounds formulated for parenteral administration, such as intravenous or intramuscular injection, other pharmaceutically acceptable forms include, *e.g.* tablets or other solids for oral administration ; liposomal formulations ; time release capsules ; and any other form currently used, including cremes.

Other routes of administration are contemplated, including nasal solutions or sprays, aerosols or inhalants, or vaginal or rectal suppositories and pessaries.

In certain embodiments, the use of liposomes and/or nanoparticles is contemplated for the introduction of inhibitory antibodies or other agents into host cells. The formation and use of liposomes is generally known to those of skill in the art, and is also described below.

Nanocapsules can generally entrap compounds in a stable and reproducible way. To avoid side effects due to intracellular polmeric overloading, such ultrafine particles (sized around 0.1 µm) should be designed using polymers able to be degraded in vivo. Biodegradable polyalkylcyanoacrylate nanoparticles that meet these requirements are contemplated for use in the present invention, and such particles may be are easily made.

Liposomes are formed from phospholipids that are dispersed in an aqueous medium and spontaneously form multilamellar concentric bilayer vesicles (also termed multilamellar vesicles (MLVs)). MLVs generally have diameters of from 25 nm to 4 µm. Sonication of MLVs results in the formation of small unilamellar vesicles (SUVs) with diameters in the range of 200 to 500 Å, containing an aqueous solution in the core.

The following information may also be utilized in generating liposomal formulations. Phospholipids can form a variety of structures other than liposomes when dispersed in water, depending on the molar ratio of lipid to water. At low ratios the liposome is the preferred structure. The physical characteristics of liposomes depend on pH, ionic strength and the presence of divalent cations. Liposomes can show low permeability to ionic and polar substances, but at elevated temperatures undergo a phase transition which markedly alters their permeability. The phase transition involves a change from a closely packed, ordered structure, known as the gel state, to a loosely packed, less-ordered structure, known as the fluid state. This occurs as a characteristic phase-transition temperature and results in an increase in permeability to ions, sugars and drugs.

Liposomes interact with cells via four different mechanisms : endocytosis by phagocytic cells of the reticuloendothelial system such as macrophages and neutrophils ; adsorption to the cell surface, either by non-specific weak hydrophobic or electrostatic forces, or by specific interactions with cell-surface components ; fusion with the plasma cell membrane by insertion of the lipid bilayer of the liposome into the plasma membrane, with simultaneous release of liposomal contents into the cytoplasm ; and by transfer of liposomal lipids to cellular or subcellular membrane, or *vice versa,* without any association of the liposome contents. Varying the liposome formulation can alter which mechanism is operative, although more than one may operate at the same time.

If a stroke occurs, BARD1 inhibition can be useful to limit injury to the brain. The ideal mode of administration of antisense nucleic acids or other inhibitory agents is by intraperitoneal (i.p.) or intravenous (i.v.) injection at a dose of about 0.5-20 mg/kg, or alternatively of about 1-1000 µg/kg. Subcutaneous or intraarterial injection into the carotid artery, or direct injection into the brain, *e.g.* intracerebroventricular injection for dispersion into other areas, are also contemplated.

The time of administration of a dose of inhibitory agent is preferably up to about four hours after onset of an ischemic episode. However, therapeutic intervention can also be useful concurrently with the onset of an ischemic episode or even prior to onset of ischemia.

The below figures and examples illustrate the invention without limiting its scope.

### DESCRIPTION OF THE FIGURES :

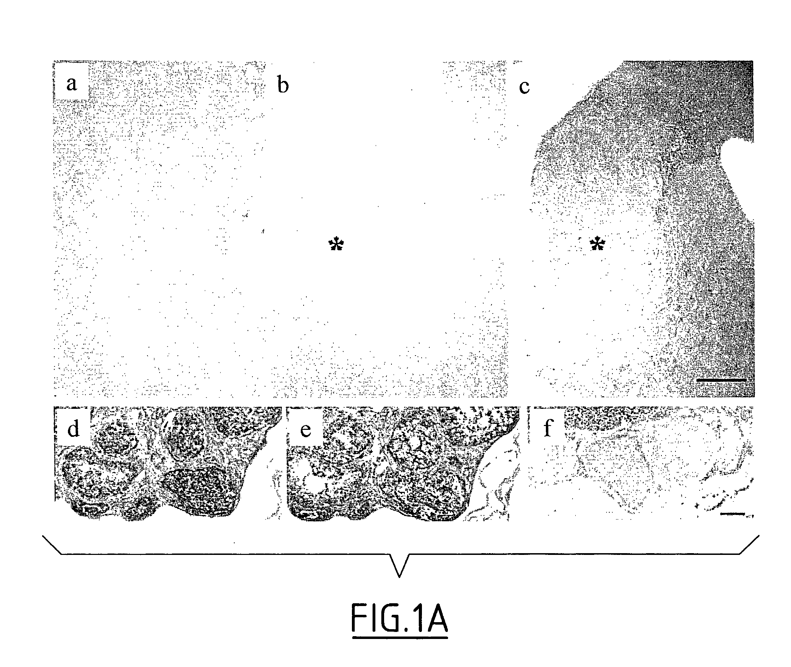

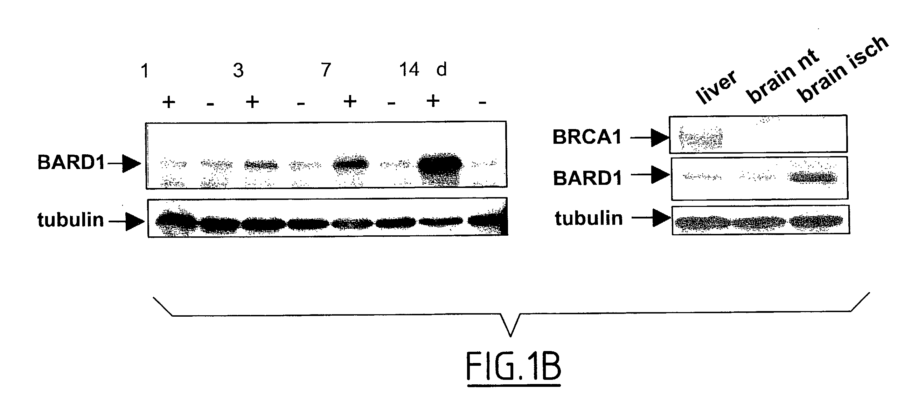

**Figure 1.** BARD1 expression is upregulated upon stress.
   (**A**) BARD1, but not BRCA1, is upregulated in a mouse stroke model. In brains from untreated animals, neither BARD1 (***a***) nor BRCA1 was detected by immunocytochemistry. As shown at day 7 after MCAO, BARD1 was not detected in the infarct core region (*), but in the penumbra (***c***); staining remained negative for BRCA1 (***b***). Similar results were obtained at day 3 and 14. Bar, 550 µm. Unlike the expression in brain BRCA1 **(*d*)** and BARD1 (e) are expressed in the mouse ovary. Primary antibody was omitted in **(*f*).** Bar, 100 µm. **(B)** As assessed by Western blotting, an increase of BARD1 can be observed from day 1 through day 14 after MCAO, in extracts from the treated hemisphere (+) but not in the untreated hemisphere (-) (left panel). Expression of BARD1 and BRCA1 was compared on liver and non treated (nt) or ischemic (isch) brain tissue (right panel). For both immuno-cytochemistry and Western blotting, the BARD1 antibody N-19 (Santa Cruz sc-7373) was used. The same staining was observed with a C-terminal anti-BARD1 antibody (Santa Cruz sc-7372). Anti-BRCA1 antibody was M-16 (Santa Cruz). Antibody preabsorption with specific peptides (Santa Cruz) or omission of primary antibody resulted in absence of the signal (not shown).

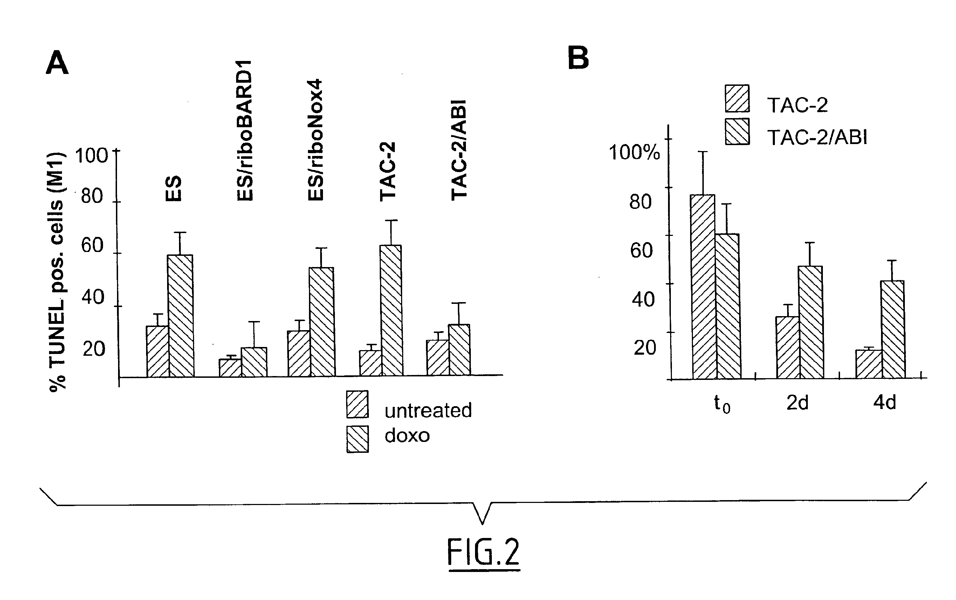

**Figure 2.** Repression of BARD1 reduces the apoptotic response to genotoxic stress.
   BARD1 repression was achieved by stable transfection of BARD1 ribozyme in ES cells (ES riboBARD1) or a BARD1 antisense in murine mammary cells (TAC-2/ABI). After treatment with doxorubicin (5 µg/ml, 6h), apoptosis was monitored by flow cytometric analysis of TUNEL staining. Comparison of results obtained for ES, ES/riboBARD1, TAC-2, TAC-2/ABI, or ES/riboNox4 (transfected with an unrelated ribozyme) is shown in (A) with mean x (TUNEL-fluorescence) ± S.E. (n=3). Cell numbers of TAC-2 cells, or BARD1-repressed TAC-2/ABI cells in the absence or presence of doxorubicin (10 µg/ml), were monitored after two and four days (**B**). Five fields of cells from each time point and each cell type were analyzed and the mean ± S.E. remaining adherent cells are shown (**B**).

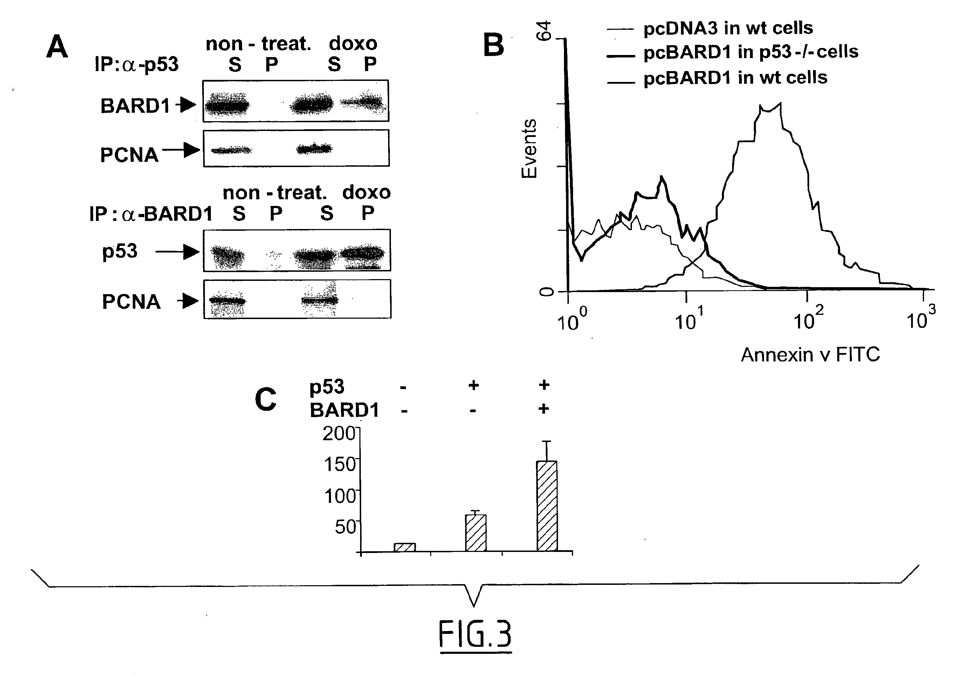

**Figure 3.** BARD1 induces apoptosis through interaction with functional p53.
   BARD1-p53 interaction was assayed by co-immunoprecipitation **(A).** Anti-p53 (agarose-coupled, Santa Cruz) or anti-BARD1 antibodies (N-19, Santa Cruz) were used for immunoprecipitations (IP) from non-treated (non-treat) and doxorubicin treated (doxo) cells, and Western blots were incubated with anti-BARD1 (PVC, Irminger-Finger et al., 1998) and anti-PCNA (Santa Cruz) or anti-p53, respectively. Supernatants (S) and pellet (P) fractions are presented. Western blots were probed with anti-p53 and anti-BARD1. BARD1 induced apoptosis depends on functional p53, as demonstrated by annexin V staining (B) of wild type or p53 -/- ES cells transiently transfected with pcDNA3 or BARD1. BARD1 is required for p53 stabilization through protein-protein interaction as demonstrated by cotransfection of BARD1 and p53 in p53 deficient ES cells (C), p53-/- ES cells were transfected with p53 or BARD1 plus p53. Increase of p53 was monitored on Western blots, and p53 protein bands were quantified. Bar graph presents intensities of band as arbitrary units.
**Figure 4.** Model of dual functionality of BARD1. Increase of BARD1 upon stress: pathway (1) BRCA1-BARD1 heterodimer formation, observed as nuclear dots (Scully et al., 1997), induction of DNA repair functions; pathway (2) BARD1 excess over BRCA1 (possibly as homodimers), BARD1 interaction with p53, affecting p53 stability and induction of apoptosis.

### EXAMPLES

### I - Experimental Procedures

### Cerebral ischemia

Cerebral ischemia was induced in the right brain hemisphere of C57BL/6 mice by medium cerebral artery occlusion (MCAO) as described (Martinou et al., 1994). Mice were sacrificed at 1, 3, 7, and 14 days after MCAO and their brains were prepared for immunohistochemistry and Western blots. Protein extracts from brain tissue of the right and left hemisphere were prepared separately.

### Western blotting and immunoprecipitation

For Western blots, protein extracts from tissues or cell cultures were prepared in standard lysis buffer (150 mM NaCI; 0.1% SDS; 50 mM Tris pH 8.0; 2 mM EDTA pH 8.0) and analyzed by Western blotting using anti-p53 (Santa Cruz FL-393), anti-BARD1 PVC (Irminger-Finger et al., 1998), N-19 and C-20 (Santa-Cruz sc-7373), or anti-β-tubulin (Boehringer) antibodies. Immuno precipitation was performed by homogenizing cell samples in 100 µl (100-150 µg total protein) RIPA buffer (150 mM NaCI; 1% NP-40; 0.5% NaDOC; 0.1% SDS; 50 mM Tris pH 8.0; 2 mM EDTA pH 8.0). Cell extracts were incubated with agarose-coupled p53 antibodies (Fl 393 Santa Cruz) in a 1 to 100 dilution for 2 hours, or BARD1 N-19 antibodies in 1 to 100 dilution over night, followed by incubation with sepharose-bound protein G for 2 hours. Extracts were centrifuged, pellets were washed twice with RIPA buffer and resuspended in 100 µl RIPA buffer. 25 µl of supernatant and pellet fractions were analyzed by Western blotting. Total protein content in supernatant and pellet fractions was compared by PonceauS staining after protein transfer and was 90 to 10 percent at least. Antibody reactive bands were visualized, scanned and quantified using Imagequant.

### Doxorubicin treatment and UV exposure

TAC-2, ES, or HeLa cells were grown to sub-confluence in 100 mm Petri dishes, exposed to UV light to reach 2.5 to 10 Jm⁻² and cultured for another 3 hours at 37°C before harvesting. Doxorubicin (10, 50, 100 µg/ml) was added to the culture medium for 6 to 12 hours before harvesting for apoptosis tests.

### Flow cytometry

Cells were permeabilized and fixed with CytoFix (Pharmingen), incubated with anti-BARD1 antibodies and FITC-coupled secondary antibodies for flow cytometric analysis on a Galaxy Pro flow cytometer (Dako, Switzerland).

### Generation of expression clones and cell lines

BARD1 expression clone was produced by cloning the coding sequence of the mouse BARD1 cDNA (Irminger-Finger et al., 1998) into the Kpnl and Notl sites of pcDNA3. The Q564H mutation was generated with Quick Change Site-directed mutagenesis kit (Stratagene). The murine BRCA1 cDNA, a generous gift of G. Lenoir, was cloned into the pcDNA3 vector.

Transfections were performed reproducibly with transfecten (Qiagen). 1 to 4 µg of DNA were transfected per 100 mm Petri dish. Transfection efficiency was between 20 and 30 percent as assayed by parallel transfection of GFP cloned into pcDNA3 and analyzed by flow cytometry and fluorescence microscopy.

### Generation of BARD1 antisense and ribozyme cell lines

The ribozyme-carrying mouse ES cells were generated by polyclonal selection for neomycin resistance, using a BARD1 specific ribozyme (Irminger-Finger et al., 1998). As control for non-specific ribozyme effects, mouse ES cells were transfected with an irrelevant ribozyme (riboNox4). The mouse mammary gland cell clone TAC-2/ABI, which expresses BARD1 antisense RNA (Irminger-Finger et al., 1998) also reduces BARD1 expression by 50 to 60 percent. The p53-deficient ES cells were described before (Lakin et al., 1999). Brca1-deficient ES cells were described in Aprelikova et al., 2001.

### RT-PCR

Total RNA was prepared from cell cultures with Qiagen purification kits. RT-PCR (Perkin Elmer) was performed in simultaneous one step or in two steps reactions, leading to identical results. BARD1 primers were designed to amplify region 1840 through 2360 of the BARD1 cDNA, and p53 primers were designed to amplify region 118 through 310 of the p53 cDNA sequence. The p53 coding sequence was cloned by RT-PCR from TAC-2 cells (Genbank accession #AY044188).

### Quantification of apoptosis

Apoptosis was monitored by several criteria, including changes in cellular morphology observed by light microscopy and nuclear morphology observed after DAPI staining; DNA laddering was performed with Quick Apoptotic DNA Laddering Kit (Biosource International), and annexin V staining and TUNEL assays were performed with kits from Molecular Probes, and analyses were carried out on DAKO Galaxy pro flow cytometer. Caspase 3 activity test were performed with kits from (Molecular Probes) and analyzed on a multi-label counter Victor from Perkin Elmer (Switzerland).

### II - Results

### BARD1 upregulation in response to hypoxia in vivo

To investigate a putative role of BARD1 in cell death, the inventors used a well established mouse stroke model (Martinou et al., 1994). To induce ischemic stroke, median cerebral artery occlusion (MCAO) was performed in the right brain hemisphere. Necrotic cell death occurs during the first few days in the stroke region, while apoptosis develops over several weeks in the surrounding tissue, the penumbra (Martinou et al., 1994). In the brain of untreated animals, no BARD1 was found (Fig. 1Aa), consistent with the previously described absence of BARD1 mRNA in the central nervous system (Ayi et al., 1998). Positive BARD1 immunostaining was detected after ischemia predominantly in the penumbra (Fig. 1Ac), increasing from day 1 up to day 14 in the ischemic hemisphere (Fig. 1B, left panel). In contrast, BRCA1 was not detected in the brain neither of untreated animals nor after MCAO (Fig. 1Ab, Fig. 1B, right panel). As positive control for the BRCA1 antibody sections of mouse ovarian tissue were analyzed and showed expression of BARD1 and BRCA1 in ovarian follicles (Fig. 1Ad-f). These results suggest that the role of BARD1 in hypoxia-induced brain damage is independent of BRCA1. BARD1 induction in ischemia would be compatible with its involvement in cellular stress response and possibly the induction of apoptosis. To further clarify the potential role of BARD1 in apoptosis, the inventors proceeded with *in vitro* studies.

### Repression of BARD1 reduces apoptotic response to genotoxic stress

The inventors studied the effect of BARD1 repression. For this purpose they used two different systems for BARD1 repression, ribozymes and antisense RNA. Mouse ES cells stably expressing anti-BARD1 ribozymes were generated, and TAC-2 cells stably expressing BARD1 antisense RNA were described previously (TAC-2/ABI; Irminger-Finger et al., 1998). In both systems, doxorubicin induced much less apoptosis (i.e. TUNEL-positive cells), as compared to control cells or cells transfected with an irrelevant ribozyme (Fig. 2A). Resistance to doxorubicin (10 µg/ml) induced apoptosis through BARD1 antisense expression could also be monitored by simple cell count (Fig. 2B) or by measuring caspase 3 activity (Molecular Probes). In control cells doxorubicin led to almost 100 percent cell death within a few days, whereas BARD1-repressed cells showed delayed and reduced response. Taken together, these results suggest that the apoptotic response to doxorubicin is, at least in part, mediated through BARD1.

### BARD1-p53 interaction is required for BARD1 induced apoptosis

To determine how BARD1 induces p53 accumulation, we investigated whether the two proteins interact physically by performing co-immunoprecipitation experiments (Fig. 3A). Cell lysates from TAC-2 cells (Fig. 7A-C), mouse ES cells, or HeLa cells (not shown), untreated or after treatment with doxorubicin, were tested. When using either anti-BARD1 antibodies or anti-p53 antibodies, BARD1 and p53 co-immunoprecipitated. Using p53 antibodies, 36 percent (+/- 4; n=2) of BARD1 were found in the pellet after treatment with doxorubicin (Fig. 3A, upper panel) and p53 co-immunprecipitation with BARD1 antibodies resulted in 52 percent (+/- 3; n=2) of the protein in the pellet fraction after doxorubicin treatment (Fig. 3A, lower panel). The nuclear protein PCNA, shown previously to be associated with BRCA1 in DNA repair functions (Scully et al. 1997) did not co-immunoprecipitate under our experimental conditions, demonstrating specificity of the p53-BARD1 interaction. However, this co-immunoprecipitation was observed only in doxorubicin-treated cells. The lack of co-immunoprecipiation of BARD1 and p53 in non-treated cells may simply be due to the very low p53 protein levels in non-treated cells. The interaction between BARD1 and p53 was also observed in TAC-2 cells in which p53 elevations were induced by BARD1 transfection). Efficiency of BARD1-p53 interaction is compared in transient transfections of BARD1 or BARD1Q564H (BARD1Q>H) mutant. Co-immunoprecipitation was markedly decreased for the BARD1 mutant Q564H. Only 19 percent (+/- 2; n=3) of p53 co-immunprecipitated with BARD1 Q564H, while cells transfected with wild type BARD1 showed 58 percent of p53 protein in the pellet fraction. Thus, the tumor-associated BARD1 Q564H mutant interacts less avidly with p53 than wild type BARD1.

To further ascertain the role of p53 in BARD1-induced apoptosis, the inventors investigated the apoptotic activity of BARD1 in p53-deficient mouse ES cells (Corbet et al., 1999). Transfection of BARD1 into p53-/- cells did not lead to apoptosis, as determined by annexin V staining (Fig. 3B) and DNA ladder assay, while apoptosis was induced in the corresponding wild type cells. Thus, p53 is required for BARD1-induced apoptosis.

The data presented so far demonstrate that increase of BARD1 leads to an increase of p53 protein which is not accompanied by detectable elevations of p53 mRNA. Together with the direct protein-protein interaction of the two molecules shown above, this suggests postranslational stabilization of p53 by BARD1. To further substantiate this point, p53-deficient ES cells were transfected with vector sequence, with p53 plus control vector, or with p53 plus BARD1. In this system p53 (expressed from the constitutive CMV promoter) cannot be controlled on a transcriptional level, but p53 protein stabilization must result from interaction with BARD1. Indeed, as shown in Fig. 3C, markedly higher levels of p53 protein are observed in cells co-transfected with p53 and BARD1, than in cells transfected with p53 only.

### REFERENCES

Abbott et al., (1999) J. Biol. Chem. 274, 18808-18812
Akli et al. (1993) Nature Genetics 3 224
Aprelikova et al., (2001) J. Biol. Chem. 276(28):25647-50.
Ayi T. C., Tsan J. T., Hwang L. Y., Bowcock A. M., Baer R. (1998). Conservation of function and primary structure in the BRCA1-associated RING domain (BARD1) protein. Oncogene 17, 2143-2148.
Bienstock R. J., Darden T., Wiseman R., Pedersen L., Barrett J. C. (1996). Molecular modeling of the amino-terminal zinc ring domain of BRCA1. Cancer Res. 56, 2539-2545.
Chen et al. (1995) Science, 270, 789-791
Chiocca et al. (1990) New Biol. 2 739
Corbet S. W., Clarke A. R., Gledhill S., Wyllie A.H. (1999). P53-dependent and -independent links between DNA-damage, apoptosis and mutation frequency in ES cells. Oncogene 18, 1537-1544.
Danos et al. (1988) PNAS 85 6460
Dobson et al. (1990) Neuron 5 353
Felgner et al. (1989) Science 337:387-388
Felgner et. al. (1987) Proc. Natl. Acad. Sci. U.S.A. 84:7413-7417
Ferenczy A. (1993). Ultrastructure of the normal menstrual cycle. Microsc Res Tech. 25, 91-105.
Gautier F., Irminger-Finger I., Gregoire M., Meflah K., Harb J. (2000). Identification of an apoptotic cleavage product of BARD1 as an autoantigen: a potential factor in the antitumoral response mediated by apoptotic bodies. Cancer Res. 60, 6895-6900.
Gowen et al, (1998) Science, 281,1009-1012
Graham et al. (1977) J. Gen. Virol. 36 59
Graham, (1984) EMBO J. 3(12) 2917
Harlow et. al. (1988)editors, Antibodies: A Laboratory Manual
Hashizume (2001) J. Biol. Chem. 276(18): 14537-40
Irminger-Finger I., Soriano J. V., Vaudan G., Montesano R., Sappino A. P. (1998). In vitro repression of Brca1-associated RING domain gene, Bard1, induces phenotypic changes in mammary epithelial cells. J Cell Biol. 143, 1329-1339.
Kleiman F. E., Manley J. L. (1999). Functional interaction of BRCA1-associated BARD1 with polyadenylation factor CstF-50. Science 285, 1576-1579.
Kleiman F. E., Manley J. L. (2001). The BARD1-CstF-50 interaction links mRNA 3'end formation to DNA damage and tumor suppression. Cell 104, 743-753.
Lakin N. D., Jackson S. P. (1999). Regulation of p53 in response to DNA damage. Oncogene. 18, 7644-7655.
Le Gal la Salle et al. (1993) Science 259 988
Levrero et al. (1991) Gene 101 195
Mackey, et al. (1988) Proc. Natl. Acad. Sci. U.S.A. 85:8027-8031
Martinou J. C., Dubois-Dauphin M., Staple J. K., Rodriguez I., Frankowski H., Missotten M., Albertini P., Talabot D., Catsicas S., Pietra C., et al. (1994). Overexpression of BCL-2 in transgenic mice protects neurons from naturally occurring cell death and experimental ischemia. Neuron 13, 1017-1030.
Meza J. E., Brzovic P. S., King M. C., Klevit R. E. (1999). Mapping the functional domains of BRCA1. Interaction of the ring finger domains of BRCA1 and BARD1. J. Biol. Chem. 274, 5659-5665.
Miki et al. (1994). A strong candidate for the breast and ovarian cancer susceptibility gene BRCA1. Science 266, 66-71.
Miyanohara et al. (1992) New Biol. 4 238
Paull et al, (2001) Proc. Natl. Acad. Sci. USA 98, 6086-6091
Perbal (1984) A practical Guide to Molecular Cloning
Roemer et al. (1992) Eur. J. Biochem. 208 211
Sambrook et al., (1989) *Molecular Cloning: A Laboratory Manual,* Second Edition Cold Spring Harbor Laboratory Press, Cold Spring Harbor, New York;
Scully et al., (1996) Science, 272, 123-126
Scully R., Chen J., Ochs R. L., Keegan K., Hoekstra M., Feunteun J., Livingston D. M. (1997 b). Dynamic changes of BRCA1 subnuclear location and phosphorylation state are initiated by DNA damage. Cell 90, 425-435.
Stratford-Perricaudet et al. (1990) Human Gene Therapy 1 241
Thai T. H., Du F., Tsan J. T., Jin Y., Phung A., Spillman M. A., Massa H. F., Muller C. Y., Ashfaq R., Mathis J. M., Miller D. S., Trask B. J., Baer R., Bowcock A. M. (1998). Mutations in the BRCA1-associated RING domain (BARD1) gene in primary breast, ovarian and uterine cancers. Hum. Mol. Genet. 7, 195-202.
Vogelstein, (1990) Nature, 348:681
Wang et al., (2000) Genes. Dev., 14, 927-939
Watt and Hogan, (2000), Science, 287:1427-1430
Wu et al. (1988 J. Biol. Chem. 263:14621-14624
Wu et al. (1992) J. Biol. Chem. 267:963-967
Wu L. C., Wang Z. W., Tsan J. T., Spillman M. A., Phung A., Xu X. L., Yang M. C., Hwang L. Y., Bowcock A. M., Baer R. (1996). Identification of a RING protein that can interact in vivo with the BRCA1 gene product. Nat. Genet. 14, 430-440.
Yoshikawa K., Ogawa T., Baer R., Hemmi H., Honda K., Yamauchi A., Inamoto T. Ko K., Yazumi S., Motoda H., Kodama H., Noguchi S., Gazdar A. F., Yamaoka Y., Takahashi R. (2000). Abnormal expression of BRCA1 and BRCA1-interactive DNA-repair proteins in breast carcinomas. Int. J. Cancer 88, 28-36.
Zhong et al, (1999) Science, 285, 747-750

## Claims

1. Use of an agent that inhibits BARD1 activity for the preparation of a medicament useful for promoting cell survival.

2. The use according to claim 1, wherein the agent inhibits BARD1 expression.

3. The use according to claim 2, wherein the agent is a BARD1 antisense nucleic acid.

4. The use according to claim 1, wherein the agent inhibits BARD1 protein functional properties.

5. The use according to claim 4, wherein the agent inhibits BARD1 interaction with p53.

6. The use according to claim 4, wherein the agent is an anti-BARD1 inhibitory antibody.

7. The use according to any of claims 1 to 6, wherein the agent that inhibits BARD1 activity is associated with an agent that inhibits p53 activity.

8. The use according to any of claims 1 to 7, wherein the agent that inhibits BARD1 activity is associated with an agent that stimulates BRCA1 expression, or with a BRCA1 expressing nucleic acid.

9. The use according to any of claims 1 to 8, wherein the medicament is useful for alleviating damage in cells subjected to ischemia.

10. The use according to claim 9, wherein ischemia results from a stroke or heart failure.

11. An *ex vivo* method for promoting cell survival, which method comprises contacting a cell with an agent that inhibits BARD1 activity.

12. A nucleic acid that comprises a BARD1 antisense sequence and a p53 antisense sequence.

13. A pharmaceutical composition containing either the nucleic acid of claim 12 or an association of
i) a BARD1 antisense nucleic acid, and
ii) a p53 antisense nucleic acid,
in a pharmaceutically acceptable carrier.

14. A kit comprising
- in a first container, a pharmaceutical composition containing a BARD1 antisense sequence in a pharmaceutically acceptable carrier; and
- in a second container, a pharmaceutical composition containing comprising a p53 antisense sequence in a pharmaceutically acceptable carrier.

15. A pharmaceutical composition comprising :
i) a BARD1 antisense nucleic acid ; and
ii) a BRCA1 encoding nucleic acid ;
in association with a pharmaceutically acceptable carrier.

16. A kit comprising :
- in a first container, a pharmaceutical composition containing a BARD1 antisense nucleic acid in a pharmaceutically acceptable carrier;
- in a second container, a pharmaceutical composition comprising an expression vector that comprises a BRCA1 encoding nucleic acid operatively associated with expression control sequences.
